# EUROPÄISCHE PATENTSCHRIFT

(11) **EP 3 157 449 B1**
(45) Veröffentlichungstag und Bekanntmachung des Hinweises auf die Patenterteilung: **18.08.2021**
(21) Anmeldenummer: 15728847.3
(22) Anmeldetag: 12.06.2015
(51) Int. Cl.: A61B 17/70, A61B 17/86, F16B 35/04

(54) **PEDIKELSCHRAUBE MIT EINSCHRAUBHILFE**
PEDICLE SCREW WITH SCREW-IN AID
VIS PÉDICULAIRE AVEC OUTIL DE VISSAGE

(30) Priorität: 20.06.2014 DE 102014108705
(43) Veröffentlichungstag der Anmeldung: 26.04.2017
(73) Patentinhaber: Aesculap AG, 78532 Tuttlingen (DE)
(72) Erfinder: KRÜGER, Sven, 78647 Trossingen (DE)
(74) Vertreter: Winter, Brandl - Partnerschaft mbB
(86) Internationale Anmeldenummer: PCT/EP2015/063140
(87) Internationale Veröffentlichungsnummer: WO 2015/193178

(56) Entgegenhaltungen:
- EP-A1- 2 258 289
- WO-A1-2012/103660
- US-A1- 2002 128 657
- US-A1- 2006 083 603
- US-A1- 2009 318 970
- US-A1- 2011 152 947
- US-A1- 2011 318 136
- US-A1- 2012 310 290
- US-A1- 2013 079 830

## Beschreibung

Die vorliegende Erfindung betrifft ein Knochenschraubensystem, insbesondere ein Pedikelschraubensystem, aufweisend eine Knochenschraube bzw. eine Pedikelschraube, eine Aufnahmehülse und eine in diese einschraubbare Klemmschraube, wobei die Aufnahmehülse eine Hülsenwand aufweist, die eine Aufnahme für einen Längsträger zur chirurgischen Verbindung benachbarter Knochen- bzw. Pedikelschrauben ausbildet und mit einem Innengewinde versehen ist, wobei die Klemmschraube mit einem Außengewinde versehen und in das Innengewinde der Aufnahmehülse einschraubbar ist.

Knochen- sowie Pedikelschrauben sind aus dem Stand der Technik bekannt. Sie dienen beispielsweise zur dorsalen Stabilisierung der Wirbelsäule mittels transpedikulärer Verschraubung. Dabei werden Pedikelschrauben in den Pedikeln jeweils benachbarter Wirbel platziert, worauf eine winkelstabile Verbindung zwischen den jeweils axial übereinander angeordneten Pedikelschrauben und einem sich axial erstreckenden Längsträger oder Steg geschaffen wird. Die Pedikelschrauben und Längsträger bilden dabei ein Wirbel-Stabilisierungssystem.

Eine Pedikelschraube weist üblicherweise einen in axialer Richtung verlaufenden Schraubenschaft mit Außengewinde auf, an den sich schraubenkopfseitig eine Aufnahmehülse, die sogenannte Tulpe, anschließt. Diese ist im Wesentlichen U-förmig ausgebildet mit gegenüberliegenden Wandabschnitten und einem dazwischen ausgebildeten, in radialer Richtung verlaufenden Spalt für den Längsträger oder Steg. In die Tulpe ist ein in axialer Richtung verlaufendes Innengewinde eingebracht. Der Längsträger wird in radialer Richtung in den Spalt der Tulpe eingelegt und mittels eines Verriegelungselements oder einer Klemmschraube, üblicherweise in Form einer Madenschraube oder Gewindemutter, das bzw. die auch als Setscrew bezeichnet wird und in das Innengewinde eingedreht ist, fixiert.

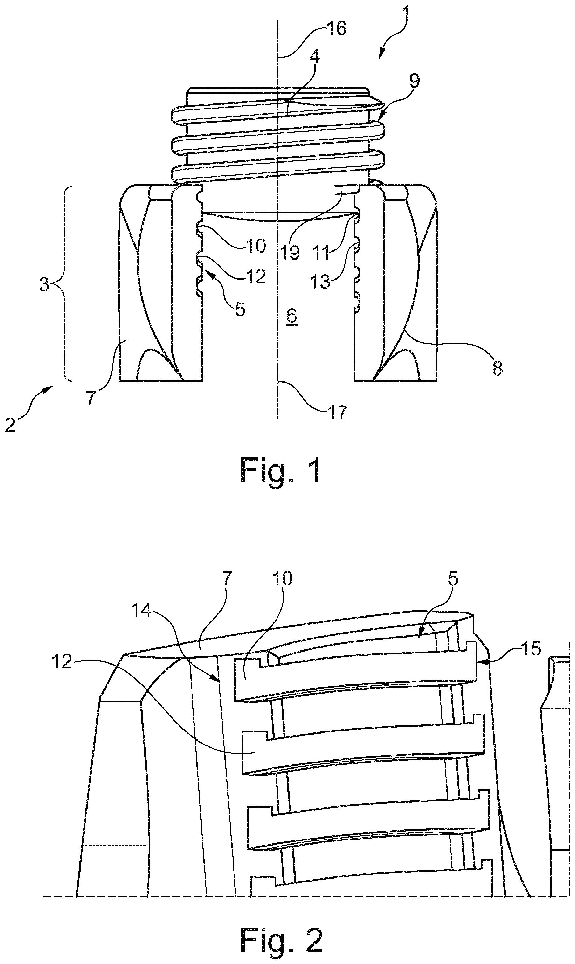

Beim Ansetzen einer Setscrew an einer Pedikelschraube besteht insbesondere bei offener Operation die Gefahr eines Verkantens infolge eines schrägen Ansetzens der Setscrew. Diese Situation entsteht insbesondere dann, wenn das Gewinde der Setscrew und das Innengewinde der Tulpe beim Ansetzen der Setscrew um ca. 180° verdreht sind, d.h. Gewindeflanke auf Gewindeflanke trifft, wie in der anliegenden Figur 1 gezeigt ist.

US 2011/0152947 A1 zeigt eine Pedikelschraube mit einer Aufnahmehülse, deren Flanken ein Innengewinde aufweisen, dessen oberster Gewindegang teilweise entfernt ist.

Bei bekannten Pedikelschrauben kann selbst bei einem korrekten Ansetzen schon leichtes Verkippen oder Verkanten der Setscrew relativ zur Achse der Pedikelschraube bzw. deren Tulpe zu einem "hackeligen" Ansetzen führen, was die Gefahr des Verkantens vergrößert. Hinzu kommt erschwerend, dass die Setscrew in der praktischen Anwendung in der Regel mit einer gewissen Kraft auf die Tulpe gedrückt wird. Diese axiale Vorkraft kann in nachteiliger Weise bereits zu einem Verkanten der Setscrew bezüglich der Tulpe führen, da die plane Oberseite der Tulpe in der Regel nicht der Gewindegeometrie der Setscrew entspricht.

Ein Verkanten kann dazu führen, dass der Gewindeanschnitt, insbesondere der der Setscrew, beschädigt wird. Schlimmstenfalls kann es zum sogenannten "crossthreading" kommen, was bedeutet, dass die Setscrew soweit zur Längsachse des Schraubenschafts und dessen Außengewindes verkantet ist, dass der Gewindeanfang der Setscrew, also der Einlaufgewindegang oder die Einlaufgewindegänge, in den falschen Gewindegang des Innengewindes der Tulpe eingreift bzw. eingreifen, was zu einer Beschädigung der Gewinde bis hin zur Unbrauchbarkeit der Setscrew und/oder der Pedikelschraube führen kann. Ein Knacken beim Ansetzen der Setscrew, welches durch das Verkanten entsteht, führt zu einer Verunsicherung des Anwenders. Dieser ist sich nicht sicher, ob das Gewinde Schaden genommen hat.

Um die vorstehend beschriebene Problematik zu vermindern sind besondere Gewindedesigns wie z.B. Rechteckgewinde oder Hinterschnittgewinde bekannt und werden als "crossthreading-reduzierend" beschrieben. Jedoch scheint ein korrektes Ansetzen der Setscrew der entscheidende Akt zur Vermeidung von Gewindebeschädigungen. Zu diesem Zweck sind Pedikelschraubensysteme bekannt, bei denen eine drehbare Führungskappe an der Setscrew montiert ist und eine zusätzliche Führung der Setscrew im Tulpenkopfgewinde ermöglicht. Es ist des Weiteren bekannt, den Gewindeeinlauf der Setscrew anzuschneiden, anders ausgedrückt in der Dicke zu reduzieren. Auf diese Weise wird ein Eingreifen des Gewindeeinlaufs der Setscrew in den Gewindeeinlauf des Tulpenkopfs erleichtert. Daneben gibt es Systeme, die eine Führung für die Setscrew bieten, um deren Verkippen gegenüber der Schraubenachse zu verhindern. Diese Führung wird anschließend abgebrochen. Beim perkutanen Arbeiten kann die angekoppelte Hülse in vielen Fällen ein Verkippen der Setscrew verhindern. Bei einem offenen Arbeiten kann ein Setscrew-Starter-Instrument mit einer Führungshülse, welche sich zum Body ausrichtet, das problematische Verkippen einschränken.

Die vorstehend beschriebenen Pedikelschraubensysteme nach dem Stand der Technik sind in nachteiliger Weise kostenintensiv und aufwendig. Sie sind in der Regel nicht in der Lage, das korrekte Ansetzen einer Setscrew an einer Pedikelschraube zu erleichtern und mit Verlässlichkeit sicherzustellen. Ein Schraubendreher mit Führungshülse kann ein Verkippen nur eingeschränkt verhindern, da diese Führungshülse für das Ankoppeln ein gewisses Spiel benötigt. Zusätzlich schränkt diese Hülse die Sicht des Arztes auf die Knochen- oder Pedikelschraube ein. Eine Optimierung des Anschnittes verbessert das Finden des Gewindes, jedoch kann es trotz dieser Maßnahme zu einem Verkanten kommen. Eine Verkanten einer "angeschnittenen" Setscrew ist kritisch, da diese durch die geringere Wandstärke sehr leicht beschädigt werden kann.

Ausgehend von dem vorstehend beschriebenen Stand der Technik liegt der Erfindung die Aufgabe zugrunde, ein Knochenschraubensystem, insbesondere ein Pedikelschraubensystem, bereit zu stellen, das das korrekte Ansetzen einer Setscrew an einer Knochen- oder Pedikelschraube erleichtert und sicherer macht, ohne dass dazu zusätzliche Elemente wie Führungshülsen etc. notwendig sind oder die Sicht des Operateurs eingeschränkt wird.

Diese Aufgabe wird nach der vorliegenden Erfindung gelöst durch ein Knochenschraubensystem bzw. Pedikelschraubensystem nach dem Oberbegriff von Anspruch 1, wobei ein Gewindegang des Innengewindes einlaufseitig einen aufgeweiteten Einlaufabschnitt aufweist, dessen Aufweitung in axialer Richtung sowie in radialer Richtung ausgebildet ist.

Die vorliegende Beschreibung erfolgt mit Bezug auf ein Knochenschraubensystem bzw. eine Knochenschraube. Die Erfindung betrifft jedoch insbesondere ein Pedikelschraubensystem bzw. eine Pedikelschraube. Die Bezeichnung Knochenschraube ist daher als auf eine Pedikelschraube gerichtet zu verstehen und umgekehrt.

Der Einlaufabschnitt ist erfindungsgemäß gegenüber dem übrigen und üblichen Verlauf des Gewindegangs aufgeweitet. Das Innengewinde der Aufnahmehülse wie auch das Außengewinde der Klemmschraube können als ein- oder mehrgängiges Gewinde ausgebildet sein. Im Falle mehrgängiger Gewinde ist es von besonderem Vorteil, wenn jeder Gewindegang einen aufgeweiteten Einlaufabschnitt aufweist und einlaufseitig aufgeweitet ist.

Durch den aufgeweiteten Einlaufabschnitt wird in vorteilhafter Weise erreicht, dass das für einen Eingriff mit einem Einlaufgewindesteg der Klemmschraube zur Verfügung stehende Querschnittsfenster gegenüber Schraubensystemen nach dem Stand der Technik vergrößert ist. Selbst im Falle eines verkanteten Ansetzens der Klemmschraube an der Aufnahmehülse, das heißt bei einem Ansetzen mit nicht fluchtenden Achsen, kann es nicht zu einem "Fressen" von Außengewinde und Innengewinde kommen. Vielmehr kann das Außengewinde der Klemmschraube ohne schädigenden Kontakt mit einer Gewindeflanke des Innengewindes in dieses eindringen. Je nach Größe der Aufweitung ist ein unschädliches Verkanten der Klemmschraube gegenüber der Aufnahmehülse mit mehr oder weniger stark abweichender Fluchtung der Achsen von Klemmschraube und Aufnahmehülse möglich. Ein besonderer Vorteil der Erfindung ist, dass sich mit wachsender Eindrehtiefe in das Innengewinde die Klemmschraube relativ zur Aufnahmehülse ausrichtet. Diese Ausrichtung erfolgt in vorteilhafter Weise automatisch und so lange, bis die Achsen von Klemmschraube und Aufnahmehülse fluchten und die Klemmschraube gerade und korrekt in dem Innengewinde sitzt. Es ist daher nicht länger erforderlich, dass ein Operateur besonderes Augenmerk auf ein unverkantetes Ansetzen der Setscrew richtet, was erhebliche Erleichterung und Zeitvorteile mit sich bringt.

Der aufgeweitete Gewindeeintritt bzw. Gewindeganganfang bildet sozusagen eine Art Rampe, um eine zunächst schief angesetzte oder beim Ansetzen verkippte Klemmschraube bzw. Setscrew bei weiteren Eindrehen in den eigentlichen Gewindegang zu führen bzw. anzuheben und dabei die Klemmschraube bzw. Setscrew wieder koaxial zum Innengewinde der Aufnahmehülse auszurichten.

Dabei ist der Einlaufabschnitt in axialer Richtung aufgeweitet. Eine derartige Aufweitung wirkt in besonders effektiver Weise gegen ein Verkanten oder Verkippen der Klemmschraube gegenüber der Achse der Aufnahmehülse. Zusätzlich ist die Aufweitung des Einlaufabschnitts in radialer Richtung ausgebildet, was eine besonders einfache Positionierung der Klemmschraube in radialer Richtung relativ zur Aufnahmehülse bewirkt. Dies ist insbesondere bei Operationen mit stark beschränkter Sicht von Vorteil.

Vorteilhafte Ausführungsformen der Erfindung sind in den Unteransprüchen beansprucht und werden nachfolgend näher erläutert.

Bei einer Ausführungsform weist der Einlaufabschnitt eine Flanke auf. Diese kann gegenüber der Flanke im weiteren Verlauf des übrigen Gewindegangs, also außerhalb des Einlaufabschnitts, in umfänglicher Richtung geneigt sein. Sie kann eine geringere Neigung als der übrige Gewindegang besitzen, kann aber auch insbesondere in das Innengewinde hinein ansteigen. Vorzugsweise handelt es sich um die zum Gewindefuß weisende Flanke des Einlaufabschnitts. Die Neigung der Flanke ist im Falle der zum Gewindefuß weisenden Flanke vorzugsweise flacher, im Falle der zum Gewindeeingang weisenden Flanke vorzugsweise steiler als die Neigung der Flanke im weiteren Verlauf des übrigen Gewindegangs. Sie kann insbesondere dem 0,5-fachen bis 0,9 fachen, bevorzugter dem 0,6-fach bis 0,8-fachen und besonders bevorzugt dem 0,7-fachen der Neigung der Flanke im übrigen Verlauf des Gewindegangs entsprechen. Im Falle der zum Gewindeeingang weisenden Flanke gelten die entsprechenden Kehrwerte. Diese Neigungen ermöglichen ein einfaches Ausrichten der Klemmschraube während des Eindrehens in das Innengewinde und vermeiden gleichzeitig eine zu starke Schwächung der Gewindeflanken sowie ein Fressen von Innen- und Außengewinde. Es ist im Rahmen der Erfindung, wenn beide Flanken des Einlaufabschnitts, also die zum Gewindefuß weisende und die zum Gewindeeinlauf weisende Flanke, in der vorgenannten Form mit abweichender Neigung ausgebildet sind. Auf diese Weise kann das Einlauffenster sehr groß ausgebildet werden.

Insbesondere kann der Querschnitt des Gewindegangs, insbesondere der Querschnitt in radialer Richtung, am Gewindeeinlauf dem 1,7-fachen bis 1,2-fachen, vorzugsweise dem 1,6-fachen bis 1,3-fachen und besonders bevorzugt dem 1,5-fachen bis 1,4-fachen des Querschnitts des Gewindegangs außerhalb des Einlaufabschnitts entsprechen. Des Weiteren kann die Dicke in axialer Richtung eines zwischen dem Einlaufabschnitt und einem benachbarten Gewindegang befindlichen Gewindestegs gegenüber der Dicke eines Gewindestegs zwischen benachbarten Gewindegängen um weniger als 50%, vorzugweise um weniger als 35% und besonders bevorzugt um weniger als 20% reduziert sein.

Der Einlaufabschnitt kann sich nach der Erfindung unterschiedlich weit in umfänglicher Richtung in das Innengewinde hinein erstrecken. Es ist bevorzugt, wenn sich der Einlaufabschnitt in umfänglicher Richtung über einen radialen Abschnitt zwischen ca. 20° und ca. 135°, vorzugsweise zwischen ca. 40° und ca. 115°, bevorzugter zwischen ca. 60° und ca. 90° erstreckt. Auf diese Weise erfolgt die letztendliche Ausrichtung der Klemmschraube erst, wenn diese für einen erforderlichen Halt im Innengewinde ausreichend weit in dieses eingeschraubt ist, so dass ein abermaliges Verkanten oder Lösen der Klemmschraube sicher vermieden werden kann. Außerdem erfolgt eine Schwächung der Tragkraft des Gewindes, wie durch Ausbilden des Einlaufabschnitts unvermeidbar ist, nur über einen geringen Teil des Gewindes, so dass mit besonderem Vorteil die Tragkraft des Gewindes nahezu unverändert bleibt.

Bei einer Ausführungsform der Erfindung ist das Innengewinde der Aufnahmehülse und/oder das Außengewinde der Klemmschraube ein Hinterschnittgewinde, insbesondere ein hinterschnittenes Sägezahngewinde auf. Der Gewindegang kann auch einen T-förmigen oder L-förmigen Querschnitt aufweisen. Dadurch kann insbesondere ein Festklemmen oder eine Selbstsicherung der Klemmschraube im Innengewinde bewirkt werden.

Bei dem Schraubensystem nach der Erfindung kann es sich um ein monoaxiales oder ein polyaxiales System handeln. Das heißt, dass die Aufnahmehülse einteilig mit der Knochenschraube ausgebildet sein kann oder dass die Aufnahmehülse als separates Element positionierbar sein kann, insbesondere winkelpositionierbar zur Knochenschraube an dieser angeordnet sein kann. Im Falle einer monoaxialen Knochenschraube ist die Aufnahmehülse fest mit deren Schaft verbunden, beispielsweise einstückig gefertigt, verschweißt oder verlötet. Im Falle einer polyaxialen Knochenschraube kann diese einen als separates Schaftbauteil gefertigten Außengewindeabschnitt mit einem kugelförmigen oder (semi)-sphärischen Schraubenkopf aufweisen. Auf diesem kann die Aufnahmehülse winkelpositionierbar angeordnet sein. Dabei kann die Aufnahmehülse den Schraubenkopf im Übergangsbereich zum Knochenschraubenschaft hintergreifen. Auf diese Weise kann die Aufnahmehülse nach einem Einschrauben der Knochenschraube in einen Knochen relativ zum Schaft verschwenkt und/oder verdreht werden, um eine gewünschte Lage und Ausrichtung im Wesentlichen unabhängig zur Ausrichtung des Schafts zu erhalten. Die Hinterschneidung verhindert dabei, dass die Aufnahmehülse vom Schaftkopf abgezogen werden kann. Anschließend kann die Aufnahmehülse mittels der Klemmschraube bei zwischengelagertem Längsträger/Steg oder mittels eines zusätzlichen Schraubelements am Schraubenkopf der Knochenschraube lagefixiert werden.

Die eingangs genannte Aufgabe wird außerdem gelöst durch ein Verfahren zur Herstellung eines erfindungsgemäßen Knochenschraubensystems, insbesondere eines Knochenschraubensystems nach einem der angehängten Ansprüche, wobei die Aufweitung im Einlaufabschnitt durch Fräsen ausgebildet wird, insbesondere indem die Aufweitung in den bereits ausgebildeten Gewindegang gefräst wird, vorzugsweise mittels eines T-Nutenfräsers.

Weitere Merkmale und Vorteile der vorliegenden Erfindung ergeben sich aus der folgenden beispielhaften und nicht beschränkenden Beschreibung der Erfindung anhand eines Pedikelschraubensystems als Beispiel für ein Knochenschraubensystem anhand von Figuren. Diese sind lediglich schematischer Natur und dienen nur dem Verständnis der Erfindung. Dabei zeigen:
Fig. 1 einen den Kopfbereich zeigenden Ausschnitt eines Pedikelschraubensystems als Beispiel für ein Knochenschraubensystem in einer seitlichen Ansicht;
Fig. 2 eine perspektivische Darstellung eines Innengewindes eines Pedikelschraubenkopfs nach dem Stand der Technik;
Fig. 3 eine perspektivische Darstellung eines Innengewindes eines Pedikelschraubenkopfs nach der Erfindung; und
Fig. 4 eine weitere Ausführungsform des Innengewindes nach der Erfindung in einer perspektivischen Darstellung.

Fig. 1 zeigt den Kopfbereich eines Pedikelschraubensystems 1 in einer seitlichen Ansicht. Das Pedikelschraubensystem 1 weist eine Pedikelschraube 2, eine Aufnahmehülse 3 sowie eine Klemmschraube 4 auf. Die Aufnahmehülse 3 kann grundsätzlich einteilig mit der Pedikelschraube 2 als sogenannte Tulpe oder als gesondertes Bauteil ausgebildet sein. Letzteres kann beweglich an der Pedikelschraube 2 angeordnet sein, so dass ein polyaxiales Pedikelschraubensystem ausgebildet ist, bei dem die Aufnahmehülse 3 relativ zur Pedikelschraube 2 winkelpositionierbar ist. Die folgende Beschreibung erfolgt mit Bezug auf eine einteilig mit der Pedikelschraube 2 ausgebildete Aufnahmehülse 3, lässt sich jedoch auch auf ein polyaxiales Pedikelschraubensystem lesen.

Die Pedikelschraube 2 ist auf der der Aufnahmehülse 3 gegenüberliegenden Seite mit einem in den Figuren nicht dargestellten Außengewinde versehen, mit dem sie in einen Pedikelkanal eines Wirbels (als Beispiel für einen Knochen) eingeschraubt werden kann. Auf der Seite der Aufnahmehülse 3 ist die Pedikelschraube 2 zu diesem Zweck mit einem in den Figuren nicht dargestellten Schraubwerkzeugeingriff versehen. Die Aufnahmehülse 3 weist eine im Wesentlichen U-förmige Gestalt mit einem in axialer Richtung darin eingebrachten und ein Innengewinde 5 aufweisenden Loch 6 auf. Anders ausgedrückt kann die Aufnahmehülse 3 ausgebildet werden, indem von einem Hohlzylinder auf radial einander gegenüberliegenden Seiten in axialer Richtung Material entfernt wird und das Loch des Hohlzylinders mit dem Innengewinde 5 versehen wird. Vom Hohlzylinder verbleiben zwei radial einander gegenüberliegenden Hülsenwandabschnitte 7, 8, deren einander zugewandte Innenflächen das Loch 6 begrenzen und mit dem Innengewinde 5 versehen sind.

Die Klemmschraube 4 ist in Form einer zu diesem Zweck üblichen Madenschraube mit einem Außengewinde 9 und einer in den Figuren nicht dargestellten stirnseitigen Werkzeugaufnahme, beispielsweise einer Innensechskantausnehmung, versehen.

Fig. 2 zeigt das Innengewinde 5 eines Pedikelschraubensystems 1 nach dem Stand der Technik. Das Innengewinde 5 ist eingängig ausgebildet. Es weist einen einzigen Gewindegang mit einem ersten Gewindeabschnitt 10 im ersten Hülsenwandabschnitt 7, einem zweiten Gewindegangabschnitt 11 im gegenüberliegenden Hülsenwandabschnitt 8, einem dritten Gewindegangabschnitt 12 im ersten Hülsenwandabschnitt 7, einem vierten Gewindegangabschnitt 13 wiederum im gegenüberliegenden Hülsenwandabschnitt 8, usw. auf. Die Gewindegangabschnitte 10, 12 im ersten Hülsenwandabschnitt 7 weisen eine Einlaufseite 14 und eine Auslaufseite 15 auf. Die Gewindegangabschnitte 11, 13 im zweiten Hülsenwandabschnitt 8 weisen ebenfalls eine Einlaufseite und eine Auslaufseite auf. Fig. 1 zeigt die Klemmschraube 4 zu Beginn des Eindrehens in die Aufnahmehülse 3. Es ist deutlich zu erkennen, dass die Klemmschraube 4 für ein korrektes Einschrauben ihres Außengewindes 9 in das Innengewinde 5 relativ zur Aufnahmehülse 3 derart auszurichten ist, dass die Achse 16 der Klemmschraube 4 mit der Achse 17 des Innengewindes 5 überdeckt. Fig. 1 zeigt eine derartige Positionierung. Es ist leicht einzusehen, dass insbesondere bei polygonalen Pedikelschrauben und/oder bei eingeschränkter Sicht und/oder schlechter Zugänglichkeit eine derartige Positionierung sehr problematisch sein kann.

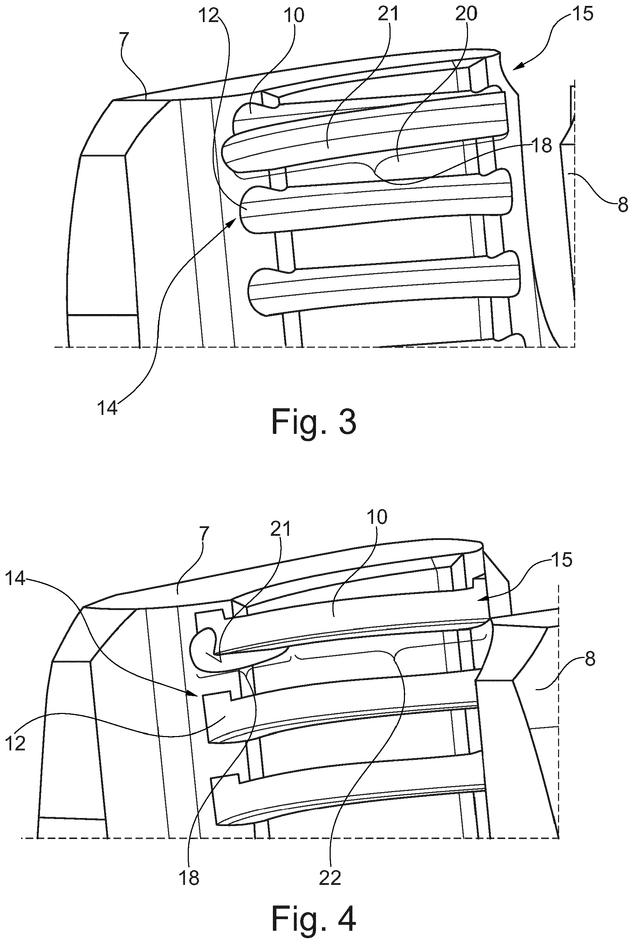

Die Figuren 3 und 4 zeigen einen erfindungsgemäß ausgebildeten Einlaufabschnitt 18 des Innengewindes 5 der Aufnahmehülse 3 in zwei unterschiedlichen Ausführungsformen. In der Ausführungsform der Fig. 3 erstreckt sich der Einlaufabschnitt 18 über die gesamte umfängliche Länge des ersten Gewindegangabschnitts 10. Bei einem Vergleich des ersten Gewindegangabschnitts 10 mit dem dritten Gewindegangabschnitt 12, der in herkömmlicher Weise ausgebildet ist, wird deutlich, dass der Querschnitt des Gewindegangabschnitts 10 in axialer Richtung einem in herkömmlicher Weise ausgebildeten ersten Gewindegangabschnitt 10 (siehe z.B. Fig. 1) aufgeweitet ist. Bei der dargestellten Ausführungsform ist nur der ersten Gewindegangabschnitt 10 derart aufgeweitet, die übrigen Gewindegangabschnitte 11, 12, 13 usw. weisen in herkömmlicher Weise einen konstanten Querschnitt auf. Durch den aufgeweiteten Einlaufabschnitt 18 wird in vorteilhafter erreicht, dass das für einen Eingriff mit einem Einlaufgewindesteg 19 der Klemmschraube 4 zur Verfügung stehende Querschnittsfenster gegenüber dem Stand der Technik vergrößert ist. Selbst im Falle eines verkanteten Ansetzens der Klemmschraube 4 an der Aufnahmehülse 3, das heißt bei einem Ansetzen mit nicht fluchtenden Achsen 16, 17, kann es nicht zu einem "Fressen" von Außengewinde 9 und Innengewinde 5, insbesondere von Einlaufgewindesteg 19 und Flanke 21 des ersten Gewindegangabschnitts 10 kommen. Vielmehr kann der Einlaufgewindesteg 19 ohne schädigenden Kontakt mit einer Gewindeflanke der ersten Gewindegangabschnitts 10 von der Einlaufseite 14 aus in diesen eindringen, und zwar je nach Größe der Aufweitung mit mehr oder weniger stark abweichender Fluchtung der Achsen 16, 17. Mit wachsender Eindrehtiefe in das Innengewinde 5 richtet sich die Klemmschraube 4 relativ zum Innengewinde 5 aus, bis die Achsen 16, 17 schließlich fluchten und die Klemmschraube gerade und korrekt in das Innengewinde 5 eingeschraubt werden kann.

Fig. 4 zeigt eine leicht abweichende Ausführungsform des aufgeweiteten Einlaufabschnitts 18. Bei dieser erstreckt sich der Einlaufabschnitt 18 weniger weit in umfänglicher Richtung in den ersten Gewindegangabschnitt 10 hinein. Des Weiteren ist die Aufweitung in axialer Richtung kleiner als bei der Ausführungsform der Fig. 3. Dies hat eine geringe Schwächung der axialen Dicke des Gewindestegs 20 an der Einlaufseite 14 zwischen dem ersten Gewindegangabschnitt 10 und dem dritten Gewindegangabschnitt 12 zur Folge. Ein weiterer Unterschied ist, das der Gewindegang des Innengewindes 5 mit einem L-förmigen Querschnitt ausgebildet ist.

### Bezugszeichenliste

- 1: Pedikelschraubensystem
- 2: Pedikelschraube, Knochenschraubeschraube
- 3: Aufnahmehülse
- 4: Klemmschraube
- 5: Innengewinde
- 6: Loch
- 7,8: Hülsenwandabschnitte
- 9: Außengewinde
- 10: erster Gewindegangabschnitt
- 11: zweiter Gewindegangabschnitt
- 12: dritter Gewindegangabschnitt
- 13: vierter Gewindegangabschnitt
- 14: Einlaufseite
- 15: Auslaufseite
- 16: Achse der Klemmschraube
- 17: Achse des Innengewindes
- 18: Einlaufabschnitt
- 19: Einlaufgewindesteg der Klemmschraube
- 20: Gewindesteg
- 21: Flanke des Einlaufabschnitts
- 22: Flanke der Gewindegangabschnitte außerhalb des Einlaufabschnitts

## Patentansprüche

1. Knochenschraube (2), insbesondere Pedikelschraube, mit einer am Schraubenkopf vorgesehenen Aufnahmehülse oder Tulpe (3), die zwei mit einem Innengewinde (5) versehene Hülsenflanken (7, 8) aufweist, zwischen welchen eine Aufnahme für einen Längsträger zur chirurgischen Verbindung benachbarter Knochenschrauben (2) ausgebildet ist, wobei ein Gewindegang (10) des Innengewindes (5) zumindest einer Hülsenflanke (7, 8) gewindeeinlaufseitig einen aufgeweiteten Einlaufabschnitt (18) aufweist,
**dadurch gekennzeichnet, dass**
die Aufweitung des Einlaufabschnitts (18) in axialer Richtung sowie in radialer Richtung ausgebildet ist.

2. Knochenschraube (2) nach Anspruch 1, **dadurch gekennzeichnet, dass** der Einlaufabschnitt (18) in axialer Richtung zum Schraubenkopf hin aufgeweitet ist.

3. Knochenschraube (2) nach Anspruch 1 oder 2, **dadurch gekennzeichnet, dass** der Einlaufabschnitt (18) eine Flanke (21) aufweist, die gegenüber der Flanke (22) im weiteren Verlauf des übrigen Gewindegangs (10, 11, 12, 13) in umfänglicher Richtung geneigt ist, insbesondere in das Innengewinde (5) hinein ansteigt.

4. Knochenschraube (2) nach Anspruch 3, **dadurch gekennzeichnet, dass** die Neigung der Flanke (21) des Gewindeeinlaufabschnitts (18) flacher ist als die Neigung der Flanke (22) im weiteren Verlauf des übrigen Gewindegangs (10, 11, 12, 13), wobei die Neigung vorzugsweise dem 0,5-fachen bis 0,9 fachen, bevorzugter dem 0,6-fachen bis 0,8-fachen und besonders bevorzugt dem 0,7-fachen der Neigung im übrigen Verlauf des Gewindegangs (10, 11, 12, 13) entspricht.

5. Knochenschraube (2) nach einem der vorstehenden Ansprüche, **dadurch gekennzeichnet, dass** der Querschnitt (in radialer Richtung) des Gewindegangs (10) am Gewindeeinlauf (14) dem 1,7-fachen bis 1,2-fachen, vorzugsweise dem 1,6-fachen bis 1,3-fachen und besonders bevorzugt dem 1,5-fachen bis 1,4-fachen des Querschnitts des Gewindegangs (10, 11, 12, 13) außerhalb des Einlaufabschnitts (18) beträgt.

6. Knochenschraube (2) nach einem der vorstehenden Ansprüche, **dadurch gekennzeichnet, dass** sich der Einlaufabschnitt (18) in umfänglicher Richtung über einen radialen Abschnitt zwischen ca. 20° und ca. 135°, vorzugsweise zwischen ca. 40° und ca. 115°, bevorzugter zwischen ca. 60° und ca. 90° erstreckt.

7. Knochenschraube (2) nach einem vorstehenden Ansprüche, **dadurch gekennzeichnet, dass** das Innengewinde (5) ein Hinterschnittgewinde, insbesondere ein hinterschnittenes Sägezahngewinde ist oder der Gewindegang (10, 11, 12, 13) einen T-förmigen oder L-förmigen Querschnitt aufweist.

8. Knochenschraube (2) nach einem der vorstehenden Ansprüche, **dadurch gekennzeichnet, dass** die Dicke in axialer Richtung eines zwischen dem Einlaufabschnitt (18) und einem benachbarten Gewindegang (12) befindlichen Gewindestegs (20) gegenüber der Dicke eines Gewindestegs (20) zwischen benachbarten Gewindegängen (10, 11, 12, 13) um weniger als 50%, vorzugweise um weniger als 35% und besonders bevorzugt um weniger als 20% reduziert ist.

9. Knochenschraube (2) nach einem der vorstehenden Ansprüche, **dadurch gekennzeichnet, dass** die Aufnahmehülse 3 einteilig mit der Knochenschraube ausgebildet ist oder das die Aufnahmehülse 3 als separates Element positionierbar, insbesondere winkelpositionierbar zur Knochenschraube 2 an dieser angeordnet ist.

10. Verfahren zur Herstellung einer Knochenschraube (2) nach einem der vorstehenden Ansprüche, **dadurch gekennzeichnet, dass** die Aufweitung im Einlaufabschnitt (18) durch Fräsen ausgebildet wird, insbesondere indem die Aufweitung in den bereits ausgebildeten Gewindegang (10) gefräst wird, vorzugsweise mittels eine T-Nutenfräsers.

## Claims

1. A bone screw (2), in particular a pedicle screw, comprising a receiving sleeve or tulip (3) provided on the screw head which includes two sleeve flanks (7, 8) provided with an internal thread (5) between which a seat for a longitudinal support for the surgical connection of adjacent bone screws (2) is formed, wherein a thread turn (10) of the internal thread (5) of at least one sleeve flank (7, 8) has a widened lead-in region (18) on the thread lead-in side,
**characterized in that**
the widening of the lead-in region (18) is formed in the axial direction as well as in the radial direction.

2. The bone screw (2) according to claim 1, **characterized in that** the lead-in region (18) is widened in the axial direction, toward the screw head.

3. The bone screw (2) according to claim 1 or 2, **characterized in that** the lead-in region (18) has a flank (21) which is inclined vis-à-vis the flank (22) in the further course of the residual thread turn (10, 11, 12, 13) in the peripheral direction and in particular rises into the internal thread (5).

4. The bone screw (2) according to claim 3, **characterized in that** the inclination of the flank (21) of the thread lead-in region (18) is flatter than the inclination of the flank (22) in the further course of the residual thread turn (10, 11, 12, 13), wherein the inclination preferably corresponds to 0.5 times to 0.9 times, more preferably to 0.6 times to 0.8 times and especially preferred to 0.7 times the inclination in the remaining course of the thread turn (10, 11, 12, 13).

5. The bone screw (2) according to one of the preceding claims, **characterized in that** the cross-section (in the radial direction) of the thread turn (10) at the thread turn (14) corresponds to 1.7 times to 1.2 times, preferably to 1.6 times to 1.3 times and especially preferred to 1.5 times to 1.4 times the cross-section of the thread turn (10, 11, 12, 13) outside the lead-in region (18).

6. The bone screw (2) according to one of the preceding claims, **characterized in that** the lead-in region (18) extends in the peripheral direction over a radial portion between approx. 20° and approx. 135°, preferably between approx. 40° and approx. 115°, more preferred between approx. 60° and approx. 90°.

7. The bone screw (2) according to one of the preceding claims, **characterized in that** the internal thread (5) is an undercut thread, in particular an undercut buttress thread or **in that** the thread turn (10, 11, 12, 13) has a T-shaped or L-shaped cross-section.

8. The bone screw (2) according to one of the preceding claims, **characterized in that** the thickness in the axial direction of a thread land (20) located between the lead-in region (18) and an adjacent thread turn (12) is reduced as compared to the thickness of a thread land (20) between adjacent thread turns (10, 11, 12, 13) by less than 50%, preferably by less than 35% and especially preferred by less than 20%.

9. The bone screw (2) according to one of the preceding claims, **characterized in that** the receiving sleeve 3 is formed integrally with the bone screw or that the receiving sleeve 3 is arranged to be positioned, in particular to be angularly positioned relative to the bone screw 2 on the latter.

10. A method of manufacturing a bone screw (2) according to one of the preceding claims, **characterized in that** the widening in the lead-in region (18) is formed by milling, in particular by milling the widening into the already formed thread turn (10), preferably by means of a T-groove cutter.

## Revendications

1. Vis à os (2), en particulier vis pédiculaire, avec une douille de réception ou tulipe (3) prévue au niveau de la tête de vis qui présente deux flancs de douille (7, 8) pourvus d'un filet intérieur (5), entre lesquels un logement pour un longeron est réalisé pour la liaison chirurgicale de vis à os contigües (2), dans laquelle un pas de filet (10) du filet intérieur (5) au moins d'un flanc de douille (7, 8) présente côté entrée de filet une section d'entrée (18) élargie,
**caractérisée en ce que**
l'élargissement de la section d'entrée (18) est réalisé dans le sens axial ainsi que dans le sens radial.

2. Vis à os (2) selon la revendication 1, **caractérisée en ce que** la section d'entrée (18) est élargie dans le sens axial vers la tête de vis.

3. Vis à os (2) selon la revendication 1 ou 2, **caractérisée en ce que** la section d'entrée (18) présente un flanc (21) qui est incliné par rapport au flanc (22) dans la suite de l'étendue du pas de filet (10, 11, 12, 13) restant dans le sens périphérique, en particulier monte dans le filet intérieur (5).

4. Vis à os (2) selon la revendication 3, **caractérisée en ce que** l'inclinaison du flanc (21) de la section d'entrée de filet (18) est plus plate que l'inclinaison du flanc (22) dans la suite de l'étendue du pas de filet (10, 11, 12, 13) restant, dans laquelle l'inclinaison correspond de préférence à 0,5 fois à 0,9 fois, de manière davantage préférée à 0,6 fois à 0,8 fois et de manière particulièrement préférée à 0,7 fois l'inclinaison dans la suite de l'étendue restante du pas de filet (10, 11, 12, 13).

5. Vis à os (2) selon l'une des revendications précédentes, **caractérisée en ce que** la section transversale (dans le sens radial) du pas de filet (10) au niveau de l'entrée de filet (14) s'élève à 1,7 fois à 1,2 fois, de préférence à 1,6 fois à 1,3 fois et de manière particulièrement préférée à 1,5 fois à 1,4 fois la section transversale du pas de filet (10, 11, 12, 13) en dehors de la section d'entrée (18).

6. Vis à os (2) selon l'une des revendications précédentes, **caractérisée en ce que** la section d'entrée (18) s'étend dans le sens périphérique sur une section radiale entre environ 20° et environ 135 °, de préférence entre environ 40° et environ 115 °, de manière davantage préférée entre environ 60° et environ 90°.

7. Vis à os (2) selon l'une des revendications précédentes, **caractérisée en ce que** le filet intérieur (5) est un filet de contre-dépouille, en particulier un filet en dent de scie contre-dépouillé ou le pas de filet (10, 11, 12, 13) présente une section transversale en forme de T ou de L.

8. Vis à os (2) selon l'une des revendications précédentes, **caractérisée en ce que** l'épaisseur dans le sens axial d'une nervure de filet (20) se trouvant entre la section d'entrée (18) et un pas de filet (12) contigu par rapport à l'épaisseur d'une nervure de pas (20) entre des pas de filet (10, 11, 12, 13) contigus est réduite de moins de 50 %, de préférence de moins de 35 % et de manière particulièrement préférée de moins de 20%.

9. Vis à os (2) selon l'une des revendications précédentes, **caractérisée en ce que** la douille de réception (3) est réalisée d'un seul tenant avec la vis à os ou la douille de réception (3) est agencée de manière positionnable comme élément séparé, en particulier de manière positionnable en angle par rapport à la vis à os (2) au niveau de celle-ci.

10. Procédé de fabrication d'une vis à os (2) selon l'une des revendications précédentes, **caractérisé en ce que** l'élargissement est réalisé dans la section d'entrée (18) par fraisage, en particulier **en ce que** l'élargissement est fraisé dans le pas de filet (10) déjà réalisé, de préférence au moyen d'une fraiseuse à rainure en T.
